# EUROPÄISCHE PATENTSCHRIFT

(11) **EP 1 360 417 B1**
(45) Veröffentlichungstag und Bekanntmachung des Hinweises auf die Patenterteilung: **30.07.2008**
(21) Anmeldenummer: 02712918.8
(22) Anmeldetag: 18.02.2002
(51) Int. Cl.: F04D 29/42, F04D 29/66, F04D 29/70

(54) **VORRICHTUNG ZUR AXIALEN FÖRDERUNG VON KÖRPERFLÜSSIGKEITEN**
DEVICE FOR AXIALLY CONVEYING BODY FLUIDS
DISPOSITIF DE TRANSPORT AXIAL DE LIQUIDES CORPORELS

(30) Priorität: 16.02.2001 DE 10108815
(43) Veröffentlichungstag der Anmeldung: 12.11.2003
(73) Patentinhaber: Berlin Heart GmbH, 12247 Berlin (DE)
(72) Erfinder: NÜSSER, Peter, 13051 Berlin (DE); MÜLLER, Johannes, 10717 Berlin (DE); PETERS, Hans-Erhard, 10437 Berlin (DE); MÜLLER, Jörg, 10439 Berlin (DE); KILIC, Ali, 10963 Berlin (DE); GRAICHEN, Kurt, 13189 Berlin (DE); RIES, Dietmar, 10247 Berlin (DE); WUNDERLICH, Klaus, 14129 Berlin (DE)
(74) Vertreter: Golkowsky, Stefan
(86) Internationale Anmeldenummer: PCT/EP2002/001739
(87) Internationale Veröffentlichungsnummer: WO 2002/066838

(56) Entgegenhaltungen:
- WO-A-00/64030
- US-A- 4 994 078

## Beschreibung

Die Erfindung betrifft eine Vorrichtung zur axialen Förderung von Körperflüssigkeiten gemäß dem Oberbegriff des Anspruches 1.

Insbesondere Körperflüssigkeiten wie Blut, die durch einen Energieeintrag irreversible Veränderungen erfahren können, wie z.B. Emulsionen und Dispersionen können beim Fördern in entsprechenden Vorrichtungen, wie Pumpen nachteiliger Weise in instabile Bereiche geraten.

Ein besonders empfindliches Fluidsystem stellt das Blut dar. Diese undurchsichtige rote Körperflüssigkeit der Wirbeltiere zirkuliert in einem in sich geschlossenen Gefäßsystem, wobei rhythmische Kontraktionen des Herzens das Blut in die verschiedenen Gebiete des Organismus hineindrücken. Hierbei transportiert das Blut die Atemgase Sauerstoff und Kohlendioxid sowie Nährstoffe, Stoffwechselprodukte und körpereigene Wirkstoffe. Das Blutgefäßsystem einschließlich des Herzens ist hierbei hermetisch von der Umwelt abgeschirmt, so dass im gesunden Organismus das Blut keine Veränderungen erfährt, wenn es über das Herz durch den Körper gepumpt wird.

Bekannt ist, daß das Blut bei Kontaktierung mit nicht körpereigenen Materialien oder durch Fremdenergieeinwirkung zur Hämolyse und Thrombenbildung neigt. Thrombenbildung kann für den Organismus tödlich sein, weil sie zu Verstopfungen im weitverzweigten Gefäßsystem führen kann. Hämolyse beschreibt den Zustand, dass über das physiologische Maß hinaus die roten Blutkörperchen innerhalb des Körpers lysiert zerstört werden. Die Ursachen für Hämolyse können mechanisch oder metabolischer Art sein. Gesteigerte Hämolyse hat multiple Organschäden zur Folge und kann bis zum Tode des Menschen führen.

Andererseits hat sich gezeigt, daß es prinzipiell möglich ist, unter bestimmten konstruktiven Voraussetzungen, die Pumpleistung des Herzens zu unterstützen bzw. sogar das natürliche Herz durch ein Kunstherz zu ersetzen. Allerdings ist ein Dauerbetrieb von implantierten Herzunterstützungssystemen oder Kunstherzen zurzeit nur begrenzt möglich, weil die Wechselwirkungen dieser Kunstprodukte mit dem Blut und dem gesamten Organismus immer noch zu nachteiligen Veränderungen des Blutes und des Organismusses führen.

Im Stand der Technik sind axiale Blutpumpen bekannt, die im Wesentlichen aus einem zylindrischen Rohr, in dem ein Förderteil, das als Rotor eines außen anliegenden Motorstators ausgebildet ist, bekannt. Der Rotor, der eine sogenannte Beschaufelung aufweist, fördert, nach dem er mittels des Motorstators in Rotation versetzt wurde, die Flüssigkeit in axiale Richtung.

So wird in der WO 00/64030 eine Vorrichtung zur schonenden Förderung von ein- oder mehrphasigen Fluiden beschrieben. Bei dieser Vorrichtung ist in Strömungsrichtung gesehen vor dem Förderteil (Rotor) eine Vorleiteinrichtung und in Strömungsrichtung gesehen nach dem Förderteil eine Nachleiteinrichtung angeordnet. Obwohl das Blut im Durchströmungsbereich der Pumpe im wesentlichen keine nachteiligen Veränderungen erfährt, hat sich jedoch nachteiliger Weise gezeigt, das sich im Zuströmbereich vor der Vorleiteinrichtung und im Abströmbereich nach der Nachleiteinrichtung der Pumpe gestörte Strömungen ausbilden können, die zu einer Veränderung des Blutes führen können.

Das US-Patent 4 994 078 zeigt eine direkt in das Herz implantierbare Blutpumpe, welche einen starren Einlasskrümmer aufweist, an welchen eine flexible Kanüle zur Anbindung an Blutgefäße angebunden ist.

Der Erfindung liegt die Aufgabe zu Grunde, den Zuströmbereich und den Abströmbereich einer Axialpumpe so auszubilden, dass auch bei einer in diesen Bereichen vorzusehenden Umlenkung der Strömung keine Strömungsablösung erfolgt, sondern ein weitgehend ungestörtes Strömungsprofil erhalten bleibt und ein optimaler operativer Einbau gewährleistet ist.

Die Lösung der Aufgabe erfolgt nach Anspruch 1.

So ist die erfindungsgemäße Vorrichtung zur axialen Förderung von Körperflüssigkeiten, enthaltend Ein- und Auslasskanülen, bestehend aus einem rohrförmigen, die Flüssigkeit im wesentlichen axial führenden Hohlkörper, in dem in axialer Ausrichtung ein mit einem außerhalb des Hohlkörpers in einem Pumpengehäuse befindlichen Motorstator in Rotation versetzbares gelagertes Förderteil angeordnet ist, wobei das Förderteil eine Rotorbeschaufelung aufweist, und wobei in Strömungsrichtung vor und nach dem Förderteil feststehende Vor- und Nachleiteinrichtungen angeordnet sind, und aus Zu- und Abströmbereiche, die am Hohlkörper strömungsrichtungsverändernd angesetzt sind, wobei im Zuströmbereich ein einen Einlasskrümmerwinkel aufweisenden am rohrförmigen Hohlkörper angesetzter Einlasskrümmer angeordnet ist, der eine Querschnittsverringerung in Richtung Vorleiteinrichtung aufweist und im Abströmbereich ein einen Auslasskrümmerwinkel aufweisenden am Hohlkörper angesetzter Auslasskrümmer angeordnet ist, und die Ein- und Auslasskrümmer einzelne Bereiche aus flexiblem Material aufweisen und die Ein- und Auslasskanäle aus flexiblem Material bestehen.

Die Ein- und Auslasskrümmer sowie die Ein- und Auslasskanülen sind vorteilhafterweise aus flexiblem Material, wobei das flexible Material im Wesentlichen aus Silikon und/oder verstärktes Silikon (Gewebe) besteht. Die Flexibilität des Materials ermöglicht einen optimalen operativen Einbau der erfindungsgemäßen Vorrichtung sowie deren Funktion im Brustraum.

Vorteilhafterweise weisen der Auslasskrümmer und der Einlasskrümmer nur einzelne Bereiche aus flexiblem Material auf.

Vorteilhafte Weiterbildungen sind in den Unteransprüchen angegeben.

So sind in Strömungsrichtung nach dem Auslasszylinder ein Strömungsdiffusor und eine Auslasskanüle angeordnet.

Der Radius des Einlasskrümmers und der Durchmesser des Eintritts des Einlasskrümmers stehen im Verhältnis 1:2.

Der Strömungsdiffusor weist in Strömungsrichtung eine Querschnittserweiterung auf.

Die Ausbildung des Zuströmbereiches und Abströmbereiches einer gattungsmäßigen Axialpumpe führt dazu, das Strömungen sanft beschleunigt werden können, ohne dass gestörte Strömungsregime entstehen. Hierbei hat sich insbesondere die Ausbildung des Einlaßkrümmerwinkels, der zwischen 45° und 50° liegt, im Verhältnis zur Veränderung des Strömungsquerschnittes als sehr vorteilhaft erwiesen. Die Größe des Auslaßkrümmerwinkels liegt hierbei zwischen 85° und 95°. Die herbeigeführte Strömungsführung kann demzufolge durch folgende vorteilhafte Eigenschaften charakterisiert werden:
- spezielle Formgebung des Einlaßkrümmers mit permanenter Beschleunigung der Strömung in der Zuströmung zur Vorleiteinrichtung
- optimierte Zuströmung zum Förderteil durch entsprechende Gestaltung der Vorleiteinrichtung
- optimierte Energieübertragung im Förderteil
- Konditionierung der Strömung und Druckrückgewinn in der Nachleiteinrichtung
- Spezielle Gestaltung des Auslaßkrümmers mit permanenter Beschleunigung der Strömung im gekrümmten Bereich
- Konditionierung der Strömung im Bereich des Verbindungsstücks zur Auslaßkanüle, daß vorzugsweise mit einer allmählichen Querschnittserweiterung in Form eines rotationssymetrischen Diffusors ausgestattet ist. Eine spezielle vorteilhafte Ausgestaltung erfährt die Vorrichtung erfindungsgemäß durch die Auswahl von elastischen hinreichend formstabilen Werkstoffen, für die Ein- und Auslaßkrümmer, die dadurch physisch bedingte permanente Bewegungen im Bereich des Anschlusses der Vorrichtung an die Herzkammer bzw. an die Aorta aufnehmen können.

Die Erfindung wird anhand einer Zeichnung im folgenden näher beschrieben. Es zeigen
- Fig. 1: eine schematische Schnittdarstellung einer gattungsgemäßen Axialpumpe mit Einlaß- und Auslaßkrümmer,
- Fig. 2: eine schematische Schnittdarstellung des Auslaßkrümmers und
- Fig. 3: eine schematische Schnittdarstellung des Einlaßkrümmers.

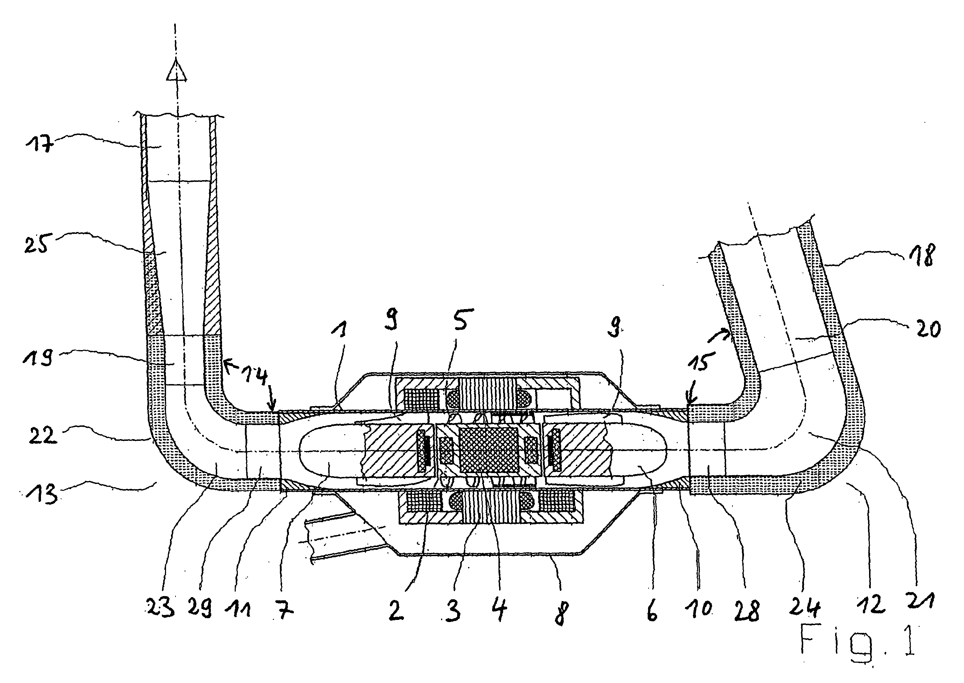

Fig. 1 zeigt eine schematische Darstellung einer gattungsgemäßen Axialpumpe mit einem Einlasskrümmer 12 und einem Auslasskrümmer 13. Der Einlasskrümmer 12 ist mit einem Einlaßansatz 28 an einem Zuströmbereich 10 eines zylindrischen Hohlkörpers 1 befestigt. Der Auslasskrümmer 13 ist mit einem Auslassansatz 29 an einem Abströmbereich 11 des zylindrischen Hohlkörpers 1 befestigt. Im zylindrischen Hohlkörper 1 befinden sich, in Strömungsrichtung gesehen, eine feststehende Vorleiteinrichtung 6 mit einer Leitbeschaufelung 9, ein schwebend gelagertes Förderteil 2, das aus einem Motorrotor 4, einer Rotorbeschaufelung 5 und einem Stützring 30 besteht. In Strömungsrichtung gesehen nach dem Motorrotor 4 ist eine feststehende Nachleiteinrichtung 7 mit einer Nachleitbeschaufelung 16 angeordnet. Der Motorrotor 4 wird mittels eines Motorstators 3, der in einem Pumpengehäuse 8 angeordnet ist, in Rotation versetzt. In einer hier beispielhaft gezeigten Axialpumpe ist am Auslasskrümmer 13 ein Strömungsdiffusor 25 vorgesehen. Zu förderndes Blut tritt über eine Einlasskanüle 18 in einen Einlasszylinder 20 des Einlaßkrümmers 12 ein und wird dann entsprechend der Krümmung 24 des Einlaßkrümmers 12 um einen Einlaßkrümmerwinkel 15 strömungsrichtungsmäßig umgelenkt und in den Einlaßansatz 28 geführt. Der Einlaßkrümmer 12 weist hier stromabwärts eine permanente Querschnittsverengung bis in den Zuströmbereich 10 auf. Das Blut wird nun über die Vorleiteinrichtung 6 am Motorrotor 4 vorbei und über die Nachleiteinrichtung 7 in den Abströmbereich 11 des zylindrischen Hohlkörpers 1 geführt. Hier tritt das Blut in den Auslaßansatz 29 des Auslaßkrümmers 13 ein und wird im Auslasskrümmer 13 strömungsrichtungsmäßig um einen Auslaßkrümmerwinkel 14 umgelenkt. Hierbei erfolgt wiederrum eine Querschnittsverengung des Auslaßkrümmers 13. Ein Auslaßzylinder 19 des Auslaßkrümmers 13 setzt hier beispielhaft an dem Strömungsdiffusor 25 an, der stromabwärts eine permanente Querschnittsvergrößerung in Richtung einer Auslaßkanüle 17 aufweist. Aufgrund der Anordnung des Einlaßkrümmers 12, der die Blutströmung unter dem Einlaßkrümmerwinkel 15 umlenkt und die Anordnung eines Auslaßkrümmers 13, der das Blut unter dem Auslaßkrümmerwinkel 14 umlenkt, wird ein ungestörter Lauf der Strömung erreicht.

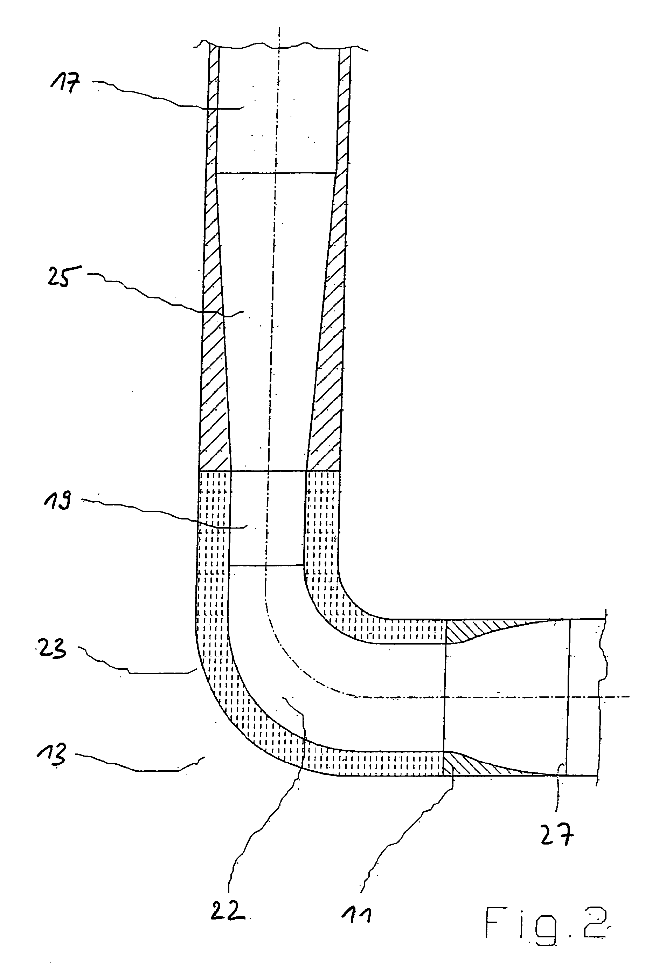

Fig. 2 zeigt in schematischer Schnittdarstellung den Auslaßkrümmer 13, der das zu fördernde Blut über einen Pumpenaustritt 27, den Abströmbereicht 11 und eine Krümmung 23 in den Auslaßzylinder 19 und dann weiter in den Strömungsdiffusor 25 und die Auslaßkanüle 17 führt. Durch diese Art der Führung der Strömung wird ein Ablösen des strömenden Blutes von einer inneren Wandung 22 des Auslaßkrümmers 13 vorteilhafterweise vermieden.

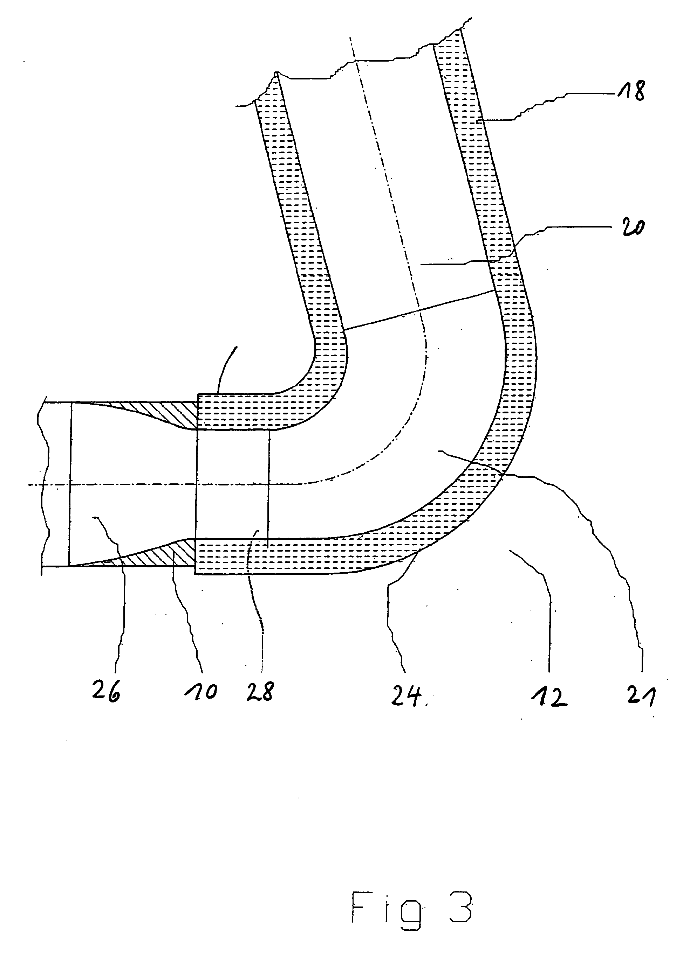

Der in Fig. 3 in schematischer Schnittdarstellung gezeigte Einlasskrümmer 12 lenkt das von der Einlaßkanüle 18 über den Einlaßzylinder 20 kommende Blut um einen Einlaßkrümmerwinkel 15 in den Einlaßansatz 28 um. Hierbei erfolgt stromabwärts eine permanente Querschnittsverengung des Einlaßkrümmers 12 bis zum Einlaßansatz 28. Über den Zuströmbereich 10 wird das Blut dann in den Pumpeneintritt 26 befördert. Aufgrund der hier erfindungsgemäß vorhandenen permanenten Strömungsquerschnittsverengung erfolgte keine Ablösung der Strömung von der Wandung 21 des Einlaßkrümmers 12.

### Bezugszeichenliste

- 1: Hohlkörper
- 2: Förderteil
- 3: Motorstator
- 4: Motorrotor
- 5: Rotorbeschaufelung
- 6: Vorleiteinrichtung
- 7: Nachleiteinrichtung
- 8: Pumpengehäuse
- 9: Leitbeschaufelung
- 10: Zuströmbereich
- 11: Abströmbereich
- 12: Einlaßkrümmer
- 13: Auslaßkrümmer
- 14: Auslaßkrümmerwinkel
- 15: Einlaßkrümmerwinkel
- 16: Nachleitbeschaufelung
- 17: Auslaßkanüle
- 18: Einlaßkanüle
- 19: Auslaßzylinder
- 20: Einlaßzylinder
- 21: Wandung
- 22: Wandung
- 23: Krümmung
- 24: Krümmung
- 25: Strömungsdiffusor
- 26: Pumpeneintritt
- 27: Pumpenaustritt
- 28: Einlaßansatz
- 29: Auslaßansatz
- 30: Stützring

## Patentansprüche

1. Vorrichtung zur axialen Förderung von Körperflüssigkeiten, enthaltend Ein- und Auslasskanülen (17, 18) und bestehend aus einem rohrförmigen, die Flüssigkeit im Wesentlichen axial führenden Hohlkörper (1), in dem in axialer Ausrichtung ein mit einem außerhalb des Hohlkörpers (1) in einem Pumpengehäuse befindlichen Motorstator (3) in Rotation versetzbares gelagertes Förderteil (2) angeordnet ist, wobei das Förderteil (2) eine Rotorbeschaufelung (5) aufweist, und wobei in Strömungsrichtung vor und nach dem Förderteil (2) feststehende Vor- und Nachleiteinrichtungen (6, 7) angeordnet sind, und aus Zu- und Abströmbereichen (10, 11) die am Hohlkörper (1) strömungsrichtungsverändernd angesetzt sind, wobei im Zuströmbereich (10) ein einen Einlasskrümmerwinkel (14) aufweisender am rohrförmigen Hohlkörper (1) angesetzter Einlasskrümmer (22) angeordnet ist, der eine Querschnittsverringerung in Richtung Vorleiteinrichtung (6) aufweist und
im Abströmbereich (11) ein einen Auslasskrümmerwinkel (15) aufweisender am Hohlkörper (1) angesetzter Auslasskrümmer (13) angeordnet ist,
**dadurch gekennzeichnet,**
**dass** die Ein- und Auslasskrümmer (12, 13) einzelne Bereiche aus flexiblem Material aufweisen und die Ein- und Auslasskanülen (17, 18) aus flexiblem Material bestehen.

2. Vorrichtung nach Anspruch 1,
**dadurch gekennzeichnet,**
**dass** in Strömungsrichtung nach dem Auslasszylinder (19) ein Strömungsdiffusor (25) und eine Auslasskanüle (17) angeordnet sind.

3. Vorrichtung nach einem der Ansprüche 1 oder 2,
**dadurch gekennzeichnet,**
**dass** das flexible Material im Wesentlichen aus Silikon und/oder verstärktem Silikon (Gewebe) besteht.

4. Vorrichtung nach einem der Ansprüche 1 bis 3,
**dadurch gekennzeichnet,**
**dass** der Auslasskrümmer (13) und der Einlasskrümmer (12) aus flexiblem Material bestehen.

5. Vorrichtung nach einem der Ansprüche 1 bis 4,
**dadurch gekennzeichnet,**
**dass** der Radius des Einlasskrümmers (12) und der Durchmesser des Eintritts des Einlasskrümmers (12) im Verhältnis 1:2 stehen.

6. Vorrichtung nach einem der vorhergehenden Ansprüche,
**dadurch gekennzeichnet,**
**dass** der Auslasskrümmer (13) in Strömungsrichtung bis zu einem Auslasszylinder (19) eine Querschnittsverringerung aufweist.

## Claims

1. A device four axially conveying body fluids, comprising inlet and outlet cannulae (17, 18) and consisting of a tubular hollow body (1), guiding the fluid substantially axially, in which in axial alignment there is arranged a mounted conveying part (2) able to be set rotating by a motor stator (3) located exterior to the hollow body (1) in a pump housing, the conveying part (2) having rotor blading (5), and fixed upstream and downstream guides (6, 7) being arranged upstream and downstream of the conveying part (2) in the direction of flow, and consisting of inflow and outflow regions (10, 11) which are attached to the hollow body (1) in a flow direction altering manner, an inlet elbow (22), which has an inlet elbow angle (14) and which is attached to the tubular hollow body (1), being arranged in the inflow region (10), which inlet elbow (22) has a cross-sectional reduction in the direction of the upstream guide (6), and an outlet elbow (13), which has an outlet elbow angle (15) and which is attached to the hollow body (1), being arranged in the outflow region (11), **characterised in that** the inlet and outlet elbows (12, 13) have individual regions of flexible material and the inlet and outlet cannulae (17, 18) are composed of flexible material.

2. A device according to claim 1, **characterised in that** a flow diffuser (25) and an outlet cannula (17) are arranged downstream of the outlet cylinder (19) in the direction of flow.

3. A device according to one of claims 1 and 2, **characterised in that** the flexible material is composed substantially of silicone and/or reinforced silicone (textile fabric).

4. A device according to any one of claims 1 to 3, **characterised in that** the outlet elbow (13) and the inlet elbow (12) are composed of flexible material.

5. A device according to any one of claims 1 to 4, **characterised in that** the radius of the inlet elbow (12) and the diameter of the entry of the inlet elbow (12) are in the ratio 1 : 2.

6. A device according to any one of the preceding claims, **characterised in that** in the flow direction, the outlet elbow (13) has a cross-sectional reduction as far as an outlet cylinder (19).

## Revendications

1. Dispositif pour l'acheminement axial de liquides corporels, comprenant des canules d'entrée et de sortie (17, 18) et composé d'un corps creux tubulaire (1) guidant le liquide de façon substantiellement axiale, dans lequel une partie d'acheminement (2) sur paliers est disposée dans une orientation axiale, cette partie pouvant être mise en rotation par un stator de moteur (3) situé en dehors du corps creux (1) dans un carter de pompe, dans lequel la partie d'acheminement (2) présente un aubage de rotor (5), et dans lequel des dispositifs de guidage stationnaires amont et aval (6, 7) sont disposés avant et après la partie d'acheminement (2) dans la direction d'écoulement, et de zones d'amenée et d'évacuation (10, 11) qui sont rapportées au corps creux (1) en modifiant la direction d'écoulement,
dans lequel, dans la zone d'amenée (10), un collecteur d'entrée (22) présentant un angle de collecteur d'entrée (14) et rapporté au corps creux tubulaire (1) est disposé qui présente une diminution de section transversale en direction du dispositif de guidage amont (6), et
dans la zone d'évacuation (11), un collecteur de sortie (13) présentant un angle de collecteur de sortie (15) et rapporté au corps creux (1) est disposé,
**caractérisé en ce que** les collecteurs d'entrée et de sortie (12, 13) présentent des zones individuelles constituées d'un matériau flexible et les canules d'entrée et de sortie (17, 18) sont constituées d'un matériau flexible.

2. Dispositif selon la revendication 1, **caractérisé en ce que** dans la direction d'écoulement après le cylindre de sortie (19), un diffuseur d'écoulement (25) et une canule de sortie (17) sont disposés.

3. Dispositif selon l'une quelconque des revendications 1 ou 2, **caractérisé en ce que** le matériau flexible se compose substantiellement de silicone et/ou de silicone renforcé (tissu).

4. Dispositif selon l'une quelconque des revendications 1 à 3, **caractérisé en ce que** le collecteur de sortie (13) et le collecteur d'entrée (12) se composent d'un matériau flexible.

5. Dispositif selon l'une quelconque des revendications 1 à 4, **caractérisé en ce que** le rayon du collecteur d'entrée (12) et le diamètre de l'entrée du collecteur d'entrée (12) présentent un rapport 1:2.

6. Dispositif selon l'une quelconque des revendications précédentes, **caractérisé en ce que** le collecteur de sortie (13) présente dans la direction d'écoulement, jusqu'à un cylindre de sortie (19), une diminution de section transversale.
